(19) Europäisches Patentamt
European Patent Office
Office européen des brevets

(11) **EP 3 188 794 B1**

(12) **EUROPÄISCHE PATENTSCHRIFT**

(45) Veröffentlichungstag und Bekanntmachung des Hinweises auf die Patenterteilung:
**28.08.2019 Patentblatt 2019/35**

(21) Anmeldenummer: **16702521.2**

(22) Anmeldetag: **29.01.2016**

(51) Int Cl.:
*A61N 1/36* (2006.01)     *A61B 5/04* (2006.01)
*A61H 23/00* (2006.01)     *A61F 7/00* (2006.01)

(86) Internationale Anmeldenummer:
**PCT/EP2016/051896**

(87) Internationale Veröffentlichungsnummer:
**WO 2016/120435 (04.08.2016 Gazette 2016/31)**

(54) **VORRICHTUNG ZUR NICHT-INVASIVEN NEUROSTIMULATION MITTELS MEHRKANAL-BURSTS**

DEVICE FOR NON-INVASIVE NEUROSTIMULATION BY MEANS OF MULTICHANNEL BURSTS

DISPOSITIF DE NEUROSTIMULATION NON EFFRACTIVE AU MOYEN DE RAFALES MULTICANAUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.01.2015 DE 102015101371**

(43) Veröffentlichungstag der Anmeldung:
**12.07.2017 Patentblatt 2017/28**

(73) Patentinhaber: **Forschungszentrum Jülich GmbH 52425 Jülich (DE)**

(72) Erfinder: **TASS, Peter Alexander 52428 Jülich (DE)**

(74) Vertreter: **Müller-Boré & Partner Patentanwälte PartG mbB Friedenheimer Brücke 21 80639 München (DE)**

(56) Entgegenhaltungen:
WO-A1-2010/043413     DE-A1-102008 039 387
DE-A1-102009 025 407     DE-A1-102010 016 461
DE-A1-102012 002 436     DE-A1-102012 002 437
US-A1- 2013 041 296     US-A1- 2013 090 519
US-A1- 2014 336 547

Printed by Jouve, 75001 PARIS (FR)

**Beschreibung**

Die Erfindung betrifft eine Vorrichtung und ein Verfahren zur nicht-invasiven Neurostimulation mittels Mehrkanal-Bursts.

Bei Patienten mit neurologischen oder psychiatrischen Erkrankungen, z. B. Morbus Parkinson, essentiellem Tremor, Dystonie, Funktionsstörungen nach Schlaganfall, Migräne, Zwangserkrankungen, Epilepsie, Tinnitus, Schizophrenie, Depression, Borderline Persönlichkeitsstörung sowie Reizdarmsyndrom, sind Nervenzellverbände in umschriebenen Bereichen des Gehirns in krankhaft synchroner Weise aktiv. In diesem Fall bildet eine große Anzahl von Neuronen synchron Aktionspotentiale aus, d. h., die beteiligten Neuronen feuern übermäßig synchron. Beim Gesunden hingegen feuern die Neuronen in diesen Hirngebieten qualitativ anders, z. B. auf unkorrelierte Weise.

Beim Morbus Parkinson verändert die pathologisch synchrone Aktivität im Thalamus und in den Basalganglien die neuronale Aktivität in anderen Hirngebieten, z. B. in Arealen der Großhirnrinde wie dem primär motorischen Cortex. Dabei zwingt die pathologisch synchrone Aktivität im Bereich des Thalamus und der Basalganglien beispielsweise den Großhirnrindenarealen ihren Rhythmus auf, so dass schließlich die von diesen Arealen gesteuerten Muskeln pathologische Aktivität, z. B. ein rhythmisches Zittern (Tremor), entfalten. Beim chronisch subjektiven Tinnitus findet sich krankhafte synchrone Aktivität in einem Netzwerk von auditorischen sowie nicht-auditorischen Hirnarealen.

Bei Patienten mit Hirnerkrankungen und Rückenmarkserkrankungen, welche durch übermäßig synchronisierte neuronale Aktivität gekennzeichnet sind, werden nicht-invasiv bestimmte raum-zeitliche Reizmuster, insbesondere die "Coordinated Reset"-Stimulation (CR-Stimulation) appliziert, um eine dauerhafte Linderung zu erzielen. Die nicht-invasive CR-Stimulation kann mittels verschiedener Stimulationsmodi realisiert werden:

(i) durch sensorische Reizung, d. h. durch physiologische Reizung von Rezeptoren, wie z. B. akustische Reizung des Innenohrs, visuelle Reizung der Retina oder mechanische (z. B. vibrotaktile) oder thermische Reizung von Haut-, Unterhaut-, Muskel- und Sehnenrezeptoren;

(ii) durch Reizung peripherer Nerven (und zugehöriger Rezeptoren) z. B. mittels elektrischem Strom (z. B. transkutane Elektrostimulation), mittels Magnetfeldern (transdermale Magnetstimulation) oder mittels Ultraschall; und

(iii) durch Reizung des Gehirns oder Rückenmarks, z. B. mittels elektrischem Strom (z. B. externe kraniale bzw. transkranielle Neurostimulation), mittels Magnetfeldern (z. B. transkranielle Magnetstimulation) oder mittels Ultraschall.

Zur Behandlung des chronisch subjektiven tonalen bzw. engbandigen Tinnitus wird die akustische CR-Stimulation verwandt. Hierzu werden Therapietöne an den dominanten Tinnituston angepasst und im Sinne der CR-Stimulation appliziert, um eine lang anhaltende, das Ausschalten der Stimulation deutlich überdauernde Desynchronisation der krankhaft synchronen Aktivität bzw. sogar eine anhaltende Desynchronisation derselben zu erzielen. Die akustische CR-Stimulation zur Behandlung des Tinnitus bewirkt eine signifikante und deutlich ausgeprägte Abnahme der Symptomatik (vgl. P. A. Tass, I. Adamchic, H.-J. Freund, T. von Stackelberg, C. Hauptmann: Counteracting tinnitus by acoustic coordinated reset neuromodulation. Restorative Neurology and Neuroscience 30, 137- 159 (2012)), eine signifikante Abnahme der krankhaften neuronalen Synchronisation in einem Netzwerk von auditorischen und nicht-auditorischen Hirnarealen (vgl. P. A. Tass, I. Adamchic, H.-J. Freund, T. von Stackelberg, C.Hauptmann: Counteracting tinnitus by acoustic coordinated reset neuromodulation. Restorative Neurology and Neuroscience 30, 137- 159 (2012); I. Adamchic, T. Toth, C. Hauptmann, P. A. Tass: Reversing patho-logically increased EEG power by acoustic CR neuromodulation. Human Brain Mapping 35, 2099-21 18 (2014)), eine signifikante Abnahme der krankhaften Interaktionen zwischen unterschiedlichen Hirnarealen im selben (vgl. A. N. Silchenko, I. Adamchic, C. Hauptmann, P. A. Tass: Impact of acoustic coordinated reset neuromodulation on effective Connectivity in a neural network of phantom sound. Neuroimage 77, 133- 147 (2013)) sowie in unterschiedlichen (vgl. I. Adamchic, B. Langguth, C. Hauptmann, P. A. Tass: Abnormal brain activity and cross-frequency cou-pling in the tinnitus network. Frontiers in Neuroscience 8, 284 (2014)) Frequenzbereichen.

DE 10 2012 002 436 A1 offenbart eine Vorrichtung zur Stimulation von Neuronen mit einer krankhaft synchronen und oszillatorischen neuronalen Aktivität mit einer nicht-invasiven Stimulationseinheit zur Applikation von Reizen, die Neuronen eines Patienten stimulieren, einer Messeinheit zum Aufnehmen von Messsignalen, die eine neuronale Aktivität der stimulierten Neuronen wiedergeben, und einer Steuer- und Analyseeinheit zur Steuerung der Stimulationseinheit und zur Analyse der Messsignale, wobei die Stimulationseinheit erste Reize appliziert, anhand der in Reaktion auf die Applikation der ersten Reize aufgenommenen Messsignale die ersten Reize ausgewählt werden, die eine Phasenrücksetzung der krankhaft synchronen und oszillatorischen neuronalen Aktivität der stimulierten Neuronen bewirken, anschließend die Stimulationseinheit die ausgewählten ersten Reize zeitversetzt appliziert, und anhand der in Reaktion auf die zeitversetzt applizierten Reize aufgenommenen Messsignale geprüft wird, ob die zeitversetzt applizierten Reize die krankhaft synchrone und oszillatorische neuronale Aktivität der stimulierten Neuronen unterdrücken.

In analoger Weise lässt sich die Parkinsonsche Erkrankung mittels vibrotaktiler CR-Stimulation behandeln.

Weitere Indikationen stellen z. B. Epilepsien, Funktionsstörungen nach Schlaganfall, chronische Schmerzsyndrome (mittels vibrotaktiler und/ oder thermischer CR- Stimulation), Migräne (z. B. mittels visueller CR-Stimulation) dar. Des Weiteren lassen sich diese Erkrankungen mit transkranieller Magnetstimulation oder direkter elektrischer Stimulation des Gehirns oder direkter Hirnstimulation mittels Ultraschall behandeln.

Für die oben aufgeführten Stimulationsmodalitäten (i) bis (iii) sollte aus den im Folgenden aufgeführten Gründen zur Vermeidung von Nebenwirkungen und/oder zur Steigerung der therapeutischen Wirksamkeit mit Reizen von geringer Intensität stimuliert werden können:

(i) Bei der sensorischen Stimulation ist es wichtig, die gewünschten Stimulationseffekte (z. B. eine Phasenrücksetzung der krankhaft synchronisierten oszillatorischen Aktivität im Gehirn bzw. Rückenmark) bei möglichst geringer Reizstärke überhaupt erzielen zu können. Z. B. müssen bei der akustischen CR-Stimulation zur Behandlung des Tinnitus typischerweise schwerhörige Patienten behandelt werden. Die Stimulation mit lauten Tönen kann das Innenohr schädigen, die Kommunikation mit anderen erschweren sowie Warnlaute (z. B. Fahrzeughupe, Fahrradklingel) verdecken bzw. vom Patienten infolge der vergleichsweise nahe an der Hörschwelle verlaufenden Unerträglichkeitsschwelle als deutlich unangenehm empfunden werden, und die laute Stimulation kann auch von der Umgebung des Patienten gehört und als störend empfunden werden. Bei der visuellen CR-Stimulation kann es insbesondere bei Migränepatienten zu unangenehmen Blendeffekten kommen. Bei der mechanischen, z. B. vibrotaktilen oder thermischen CR-Stimulation von Patienten mit chronischen Schmerzsyndromen (z. B. mit Morbus Sudeck oder Neuralgien) können schon leichtere Berührungen oder Wärmereize als unangenehm oder sogar schmerzhaft empfunden werden. Wenn in derartigen Fällen z. B. über die kontralaterale Extremität oder Gesichts- bzw. Körperhälfte behandelt werden muss, ist die Reizwirkung infolge der Applikation in der gesunden Körperhälfte nicht stark ausgeprägt. Insgesamt ist es bei der sensorischen CR-Stimulation sehr vorteilhaft, wenn mit sehr geringen Reizstärken stimuliert werden kann, da sensorische Reize (z. B. Töne, Helligkeitsschwankungen von Transmissionsbrillen etc.) die physiologische Reizverarbeitung stören können.

(ii) Um bei der elektrischen oder magnetischen Reizung peripherer Nerven möglichst fokal stimulieren zu können und Nebenwirkungen, die durch die Mitreizung benachbarter Strukturen hervorgerufen werden (z. B. Muskelkontraktionen, Schmerzempfindungen etc.), vermeiden zu können, ist es wichtig, möglichst geringe Reizstärken zu verwenden.

(iii) Sowohl die elektrische als auch die magnetische Reizung des Gehirns oder Rückenmarks sind nicht sehr fokal. Insbesondere führt die direkte elektrische Stimulation des Gehirns selbst im günstigsten Falle der Stimulation über eine Vielzahl von kleinen Elektroden und bei Verwendung aufwändiger Kopfmodelle neben einer fokalen starken Reizung zu einer Mitreizung von weit ausgedehnten Hirngebieten, die gerade bei chronischer Reizung unbedingt vermieden bzw. verringert werden sollte. In gleicher Weise sollte die Ultraschallreizung auf die tatsächlichen Zielgebiete im Gehirn beschränkt werden.

In all diesen Fällen ist es also nötig, bei möglichst geringen Reizstärken behandeln zu können, um die unerwünschte Mitreizung von Nicht-Zielarealen zu verringern. Dies allerdings führt häufig dazu, dass die Behandlung nicht hinreichend wirksam ist.

Außerdem sollte der Stimulationseffekt möglichst nachhaltig sein, um die Compliance, d. h. die Kooperativität und "Therapietreue" der Patienten sicherzustellen.

Der Erfindung liegt daher die Aufgabe zugrunde, eine Vorrichtung sowie ein Verfahren anzugeben, die es ermöglichen, selbst bei sehr geringen Reizstärken gute, robuste und insbesondere lang anhaltende therapeutische Effekte zu erzielen.

Die der Erfindung zugrunde liegende Aufgabenstellung wird durch die Merkmale der unabhängigen Ansprüche gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:

Fig. 1    eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer ersten Ausgestaltung;

Fig. 2    eine schematische Darstellung einer Stimulation mit Mehrkanal-Bursts;

Fig. 3    eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer zweiten Ausgestaltung;

Fig. 4A    eine schematische Darstellung einer Vorrichtung zur akustischen Stimulation von Neuronen mit einer krankhaft synchronen und oszillatorischen neuronalen Aktivität;

Fig. 4B    eine schematische Darstellung eines mehrere akustische Reize umfassenden Bursts;

Fig. 5    eine schematische Darstellung einer Vorrichtung zur visuellen Stimulation von Neuronen mit einer krankhaft synchronen und oszillatorischen neuronalen Aktivität; und

Fig. 6    eine schematische Darstellung einer Vorrichtung zur taktilen, vibratorischen, thermischen, transkutanen elektrischen und/oder transkutanen magnetischen Stimulation und/oder Ultraschall-Stimulation von Neuronen mit einer krankhaft synchronen und oszillatorischen neuronalen Aktivität.

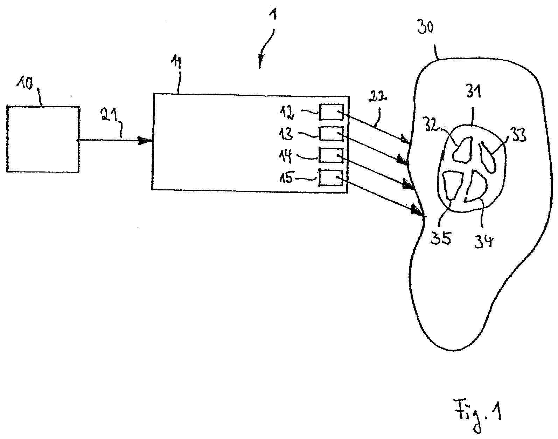

In Fig. 1 ist schematisch eine Vorrichtung 1 zur Stimulation von Neuronen mit einer krankhaft synchronen und oszillatorischen neuronalen Aktivität dargestellt. Die Vorrichtung 1 besteht aus einer Steuereinheit 10 und einer Stimulationseinheit 11, die über eine Mehrzahl von Stimulationskanälen Neuronen im Gehirn und/oder Rückenmark 30 eines Patienten stimuliert. Jeder Stimulationskanal ermöglicht die Stimulation eines anderen Zielgebiets im Gehirn und/oder Rückenmark 30 des Patienten, wobei die den Stimulationskanälen zugeordneten Zielgebiete nicht notwendigerweise disjunkt, d. h. vollständig voneinander getrennt sein müssen, sondern einander auch überlappen können. In Fig. 1 ist beispielhaft die Stimulation über vier Stimulationskanäle 12, 13, 14 und 15 dargestellt. Es kann selbstverständlich aber auch über eine andere Zahl von Stimulationskanälen stimuliert werden.

Während des Betriebs der Vorrichtung 1 führt die Steuereinheit 10 eine Steuerung der Stimulationseinheit 11 durch. Dazu erzeugt die Steuereinheit 10 Steuersignale 21, die von der Stimulationseinheit 11 entgegengenommen werden.

Die Stimulationseinheit 11 erzeugt anhand der Steuersignale 21 Reize 22 in den Stimulationskanälen 12 bis 15, die dem Patienten verabreicht werden. Die Reize 22 können sensorische Reize, z. B. akustische, visuelle, taktile, vibratorische, thermische, olfaktorische, gustatorische, transkutane elektrische, transkutane magnetische, transkranielle elektrische und/oder transkranielle magnetische Reize und/oder Ultraschall-Reize, sein. Insbesondere taktile und vibratorische Reize 22 werden auch gemeinsam appliziert und werden dann als vibrotaktile Reize 22 bezeichnet. Die Reize 22 können vom Patienten insbesondere bewusst wahrnehmbar sein. Die Parameter der Reize 22 werden von der Steuereinheit 10 eingestellt.

Die Stimulationseinheit 11 und insbesondere auch die Steuereinheit 10 sind nicht-invasive Einheiten, d. h., während des Betriebs der Vorrichtung 1 befinden sie sich außerhalb des Körpers des Patienten und werden nicht operativ in den Körper des Patienten implantiert.

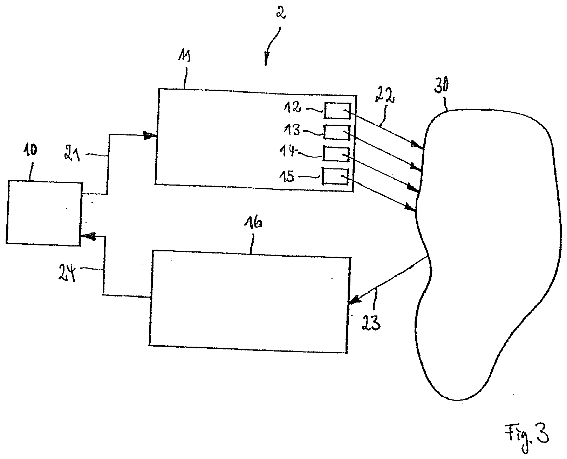

Die Vorrichtung 1 und die weiter unten, im Zusammenhang mit Fig. 3 beschriebene Vorrichtung 2 können insbesondere zur Behandlung von neurologischen oder psychiatrischen Erkrankungen eingesetzt werden, z. B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Demenzerkrankungen, Morbus Alzheimer, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bildet eine große Anzahl von Neuronen synchron Aktionspotentiale aus, d. h., die beteiligten Neuronen feuern übermäßig synchron. Hinzu kommt, dass die kranke Neuronenpopulation eine oszillatorische neuronale Aktivität aufweist, d. h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z. B. auf unkorrelierte Weise.

In Fig. 1 ist die Vorrichtung 1 während einer Stimulation von krankhaften Neuronen dargestellt. Im Gehirn und/oder Rückenmark 30 des Patienten weist mindestens eine Neuronenpopulation 31 eine wie vorstehend beschrieben krankhaft synchrone und oszillatorische neuronale Aktivität auf. Die Stimulationseinheit 11 erzeugt sensorische Reize 22, die von dem Patienten aufgenommen werden und über das Nervensystem an die krankhaft aktive Neuronenpopulation 31 im Gehirn und/oder Rückenmark 30 weitergeleitet werden. Da die krankhafte Neuronenpopulation 31 über die Stimulationskanäle 12 bis 15 an unterschiedlichen Stellen stimuliert wird, werden somit unterschiedliche Subpopulationen der Neuronenpopulation durch die Reize 22 stimuliert. In Fig. 1 sind beispielhaft vier solcher Subpopulationen 32 bis 35 dargestellt. In dem vorliegenden Ausführungsbeispiel wird die Subpopulation 32 über den Stimulationskanal 12 stimuliert, die Subpopulation 33 wird über den Stimulationskanal 13 stimuliert, die Subpopulation 34 wird über den Stimulationskanal 14 stimuliert und die Subpopulation 35 wird über den Stimulationskanal 15 stimuliert. Das jeweilige Volumen der Subpopulationen 32 bis 35 hängt von der Stärke der sie jeweils stimulierenden Reize 22 ab. Je höher die Reizstärke desto größer ist die Ausdehnung der damit stimulierten Subpopulation.

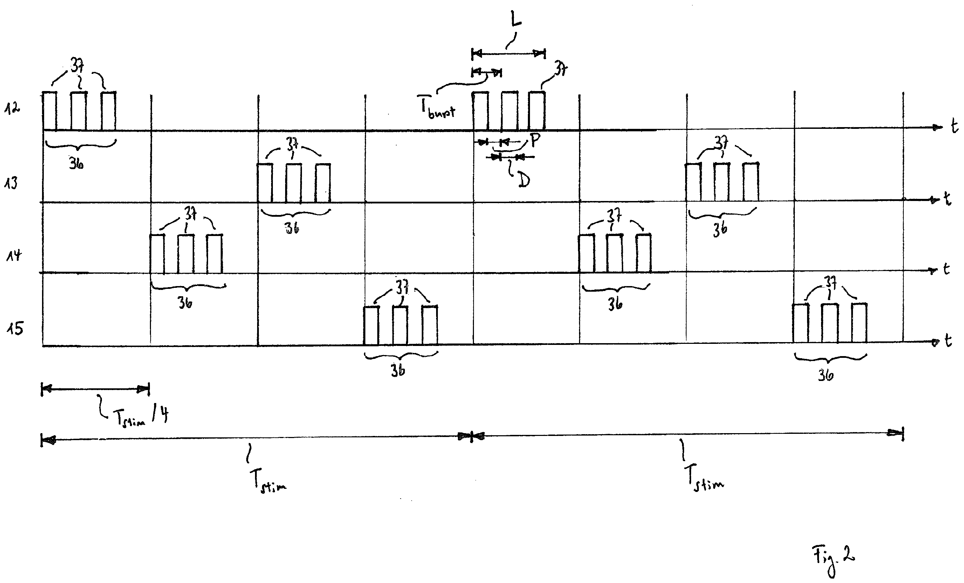

In Fig. 2 sind die von der Stimulationseinheit 11 in den Stimulationskanälen 12 bis 15 erzeugten Reize 22 in größerem Detail dargestellt. Gemäß einer Ausgestaltung werden in den Stimulationskanälen 12 bis 15 repetitiv Bursts 36 erzeugt, wobei jeder der Bursts 36 mehrere Reize 37 enthält. Bei den Reizen 37 handelt es sich jeweils insbesondere um Einzelreize oder Einzelpulse. In Fig. 2 sind untereinander die in den Stimulationskanälen 12 bis 15 erzeugten Bursts 36 gegen die Zeit t aufgetragen. Die Bursts 36 werden in einem vorgegebenen Zeitraster, das aus aufeinanderfolgenden Zyklen besteht, erzeugt. Jeder Zyklus weist eine Dauer $T_{stim}$ auf, wobei $T_{stim}$ = $1/f_{stim}$ gilt und $f_{stim}$ eine Frequenz im Bereich von 1 bis 30 Hz ist. In Fig. 2 sind beispielhaft zwei der Zyklen dargestellt. Weiterhin sind die Zyklen der Länge $T_{stim}$ in gleich lange Subzyklen unterteilt, die bei dem in Fig. 2 dargestellten Ausführungsbeispiel jeweils eine Länge von $T_{stim}/4$ aufweisen. Allgemein ist die Länge der Subzyklen gleich der Länge $T_{stim}$ geteilt durch die Anzahl der Stimulationskanäle, in denen die Bursts 36 erzeugt werden. Wird folglich über S Stimulationskanäle stimuliert, so beträgt die Dauer eines der Subzyklen $T_{stim}/S$. Das in Fig. 2 gezeigte Muster der Zyklen und Subzyklen wird periodisch fortgesetzt.

In jedem Zyklus der Länge $T_{stim}$ erzeugt die Stimulationseinheit 11 in jedem der Stimulationskanäle 12 bis 15 genau einen Burst 36, und in jedem Subzyklus der Länge $T_{stim}/4$ (bzw. $T_{stim}/S$ im allgemeinen Fall) erzeugt die Stimulationseinheit 11 in genau einem der Stimulationskanäle 12 bis 15 genau einen Burst 36. Pro Zyklus wird eine Sequenz aus vier Bursts 36 (bzw. S Bursts 36 im allgemeinen Fall) generiert, wobei jede Sequenz genau einen Bursts 36 pro Stimulationskanal enthält.

Die Reihenfolge der Stimulationskanäle 12 bis 15, in denen die Bursts 36 innerhalb einer Sequenz erzeugt werden, kann konstant sein. Alternativ kann die Reihenfolge auch nach einer bestimmten Anzahl von Zyklen variiert werden. Die Variation der Reihenfolgen kann z. B. stochastisch oder deterministisch oder gemischt stochastisch-deterministisch erfolgen. In den in Fig. 2 gezeigten Zyklen werden die Bursts 36 in den Stimulationskanälen 12 bis 15 in der Reihenfolge 12-14-13-15 erzeugt.

Zeitlich direkt aufeinanderfolgende, in unterschiedlichen Stimulationskanälen erzeugte Bursts 36 sind zeitversetzt um $T_{stim}/4$ im vorliegenden Fall mit vier Stimulationskanälen (bzw. $T_{stim}/S$ im allgemeinen Fall mit S Stimulationskanälen). Von dem Wert $T_{stim}/S$ für den Zeitversatz zwischen unmittelbar aufeinanderfolgenden Bursts 36 kann gemäß einer Ausgestaltung um z. B. bis zu ± 5%, ± 10% oder ± 20% abgewichen werden. Der Zeitversatz kann sich insbesondere auf die Anfangszeitpunkte der Bursts 36 beziehen. Solange die Reihenfolge der Stimulationskanäle 12 bis 15 innerhalb einer Sequenz konstant bleibt, werden die Bursts 36 in jedem der Stimulationskanäle 12 bis 15 periodisch mit der Frequenz $f_{stim}$ = $1/T_{stim}$ wiederholt.

Die Bursts 36 bestehen jeweils aus einer Abfolge von kurzen Reizen 37, wobei ein Burst 36 z. B. 2 bis 20, insbesondere 3 bis 9 Reize 37 enthält. Die Reize 37 können jeweils Pulse, insbesondere Einzelpulse sein. In diesem Fall können die Bursts 36 auch als Pulszüge betrachtet werden. Ein Bursts 36 kann eine Dauer L im Bereich von 50 ms bis 200 ms aufweisen. In Fig. 2 ist D die Dauer der einzelnen Reize 37, und P ist die Pause zwischen zwei innerhalb eines Bursts 36 direkt aufeinanderfolgenden Reizen 37. Die Periode, mit der die Reize 37 innerhalb eines Bursts 36 wiederholt werden, beträgt Tburst. Die Frequenz $f_{burst}$ = $1/T_{burst}$ kann im Bereich von 7 Hz bis 50 Hz liegen.

Die Dauer D und die Pause P können, müssen aber nicht gleich lang sein. Ferner können die Dauer D und die Pause P während eines Bursts 36 jeweils konstant gehalten werden, können aber auch innerhalb eines Bursts 36 oder von Burst 36 zu Burst 36 variiert werden. Z. B. können innerhalb eines Bursts 36 unterschiedlich lange einzelne Reize 37 und/oder unterschiedlich lange Pausen P zwischen den einzelnen Reizen 37 vorgesehen werden. Ferner können die Dauer D und/oder die Pause P von Burst 36 zu Burst 36 innerhalb eines Stimulationskanals und/oder zwischen Stimulationskanälen variiert werden. Die Variationen können nach deterministischen, stochastischen oder gemischt deterministischstochastischen Gesetzmäßigkeiten erfolgen.

Die dem Patienten verabreichten Bursts 36 sind so ausgelegt, dass sie in der stimulierten Neuronenpopulation 31 kein Zurücksetzen, d. h. keinen Reset, der Phase der neuronalen Aktivität der stimulierten Neuronen bewirken. Durch das Zurücksetzen würde die Phase der stimulierten Neuronen unabhängig von dem aktuellen Phasenwert auf einen oder nahe zu einem bestimmten Phasenwert, z. B. 0°, gesetzt. Bei der hier verwendeten Stimulation sind die Bursts 36 derart ausgeführt, dass eine solche Phasenrücksetzung gerade nicht bewirkt wird. Vielmehr nutzt die Erfindung ein anderes Wirkprinzip: Durch die zeitlich versetzte Reizung der jeweiligen Subpopulation werden zeitlich versetzt die Synchronisation in den jeweiligen Subpopulationen leicht erhöht. In Summe reagiert die gesamte Neuronenpopulation 31 überraschenderweise auf diese Art der Reizung, indem sie die mittlere synaptische Vernetzung kompensatorisch herunterregelt. Auf diese Weise können im Laufe der Behandlung lang anhaltende therapeutische Effekte erzielt werden.

Gemäß einer Ausgestaltung sind Zyklen vorgesehen, in denen Stimulationspausen eingehalten werden. So können während n aufeinanderfolgenden Zyklen der Länge $T_{stim}$, wie in Fig. 2 gezeigt, Bursts 36 erzeugt werden und während der darauffolgenden m Zyklen keine Bursts 36 erzeugt werden, wobei n und m nicht-negative ganze Zahlen sind. Das Muster aus n Zyklen mit Stimulation und m Zyklen ohne Stimulation kann periodisch fortgesetzt werden.

Die Periode $T_{stim}$, die zum einen die Dauer eines Zyklus und zum anderen die Periode angibt, mit der bei gleich bleibenden Sequenzen die in einem jeweiligen Stimulationskanal 12 bis 15 generierten Bursts 36 wiederholt werden, kann nahe bei der mittleren Periode der pathologischen Oszillation der Neuronenpopulation 31 mit der krankhaft synchronen und oszillatorischen neuronalen Aktivität liegen bzw. um bis zu ± 5%, ± 10% oder ± 20% von der mittleren Periode abweichen. Typischerweise liegt die Frequenz $f_{stim} = 1/T_{stim}$ im Bereich von 1 bis 30 Hz. Die Periode der pathologischen Oszillation der zu stimulierenden Neuronenpopulation 31 kann beispielsweise mittels der unten beschriebenen Messeinheit 16, insbesondere mittels EEG, gemessen werden. Dazu wird insbesondere die gemittelte dominante Frequenz der krankhaften synchronisierten oszillatorischen neuronalen Aktivität in einem insbesondere gleitenden Zeitfenster gemessen. Es ist aber auch möglich, für die Periode der pathologischen Oszillation Literatur- oder Erfahrungswerte, die sich auf die jeweilige, zu behandelnde Krankheit beziehen, zu verwenden.

Grundsätzlich kann es möglich sein, mittels der Stimulationseinheit 11 über eine beliebige Anzahl A von Stimulationskanälen zu stimulieren (A ≥ 2), jedoch müssen bei einer Stimulation nicht zwingend in allen L Stimulationskanälen Bursts 36 erzeugt werden, es können beispielsweise auch in nur einer Auswahl von B der A Stimulationskanäle die Bursts 36 erzeugt werden (2 ≤ B ≤ A).

Durch die mit der Vorrichtung 1 durchgeführte Stimulation wird trotz nur geringer Reizstärken eine Unterdrückung und insbesondere eine Desynchronisation der krankhaft synchronen und oszillatorische Aktivität der Neuronenpopulation 31 bewirkt. Eine durch die Stimulation erzielte Senkung der synaptischen Gewichte kann zu einem Verlernen der Tendenz zur Produktion krankhaft synchroner Aktivität führen. Darüber hinaus kann durch die Stimulation eine Neuorganisation der Konnektivität der gestörten neuronalen Netzwerke erzielt werden, so dass lang anhaltende therapeutische Effekte bewirkt werden können. Der erzielte synaptische Umbau ist von großer Bedeutung für die wirksame Behandlung neurologischer oder psychiatrischer Erkrankungen.

Die in Fig. 1 dargestellte Vorrichtung 1 zur Stimulation von Neuronen mit einer krankhaft synchronen und oszillatorischen neuronalen Aktivität führt eine sogenannte "open loop"-Stimulation durch, d. h., eine Stimulation ohne Sensoren, die zur Rückmeldung und/oder Steuerung der Stimulation verwendet werden.

Fig. 3 zeigt schematisch eine Vorrichtung 2 zur Stimulation von Neuronen mit einer krankhaft synchronen und oszillatorischen neuronalen Aktivität, mit der sich eine "closed loop"-Stimulation durchführen lässt. Die Vorrichtung 2 ist eine Weiterbildung der in Fig. 1 dargestellten Vorrichtung 1 und enthält genauso wie die Vorrichtung 1 eine Steuereinheit 10 und eine nicht-invasive Stimulationseinheit 11, welche die gleichen Funktionen wie die oben beschriebenen Steuer- und Stimulationseinheiten 10, 11 der Vorrichtung 1 aufweisen.

Darüber hinaus umfasst die Vorrichtung 2 eine Messeinheit 16. Der durch die Reize 22 erzielte Stimulationseffekt wird mit Hilfe der Messeinheit 16 überwacht. Die Messeinheit 16 nimmt ein oder mehrere am Patienten gemessene Messsignale 23 auf, wandelt diese gegebenenfalls in elektrische Signale 24 um und führt sie der Steuereinheit 10 zu. Insbesondere kann mittels der Messeinheit 16 die neuronale Aktivität in dem stimulierten Zielgebiet oder einem mit dem Zielgebiet verbundenen Gebiet gemessen werden, wobei die neuronale Aktivität dieses Gebiets mit der neuronalen Aktivität des Zielgebiets hinreichend eng korreliert. Mittels der Messeinheit 16 kann auch eine nicht-neuronale, z. B. muskuläre Aktivität oder die Aktivierung des autonomen Nervensystems, gemessen werden, sofern diese mit der neuronalen Aktivität des Zielgebiets hinreichend eng korreliert sind.

Die Messeinheit 16 enthält ein oder mehrere Sensoren, die es insbesondere ermöglichen, eine Ab- bzw. Zunahme der Amplitude der pathologischen oszillatorischen Aktivität nachzuweisen.

Als Sensoren können nicht-invasive Sensoren eingesetzt werden, z. B. chronisch oder intermittent genutzte Elektroenzephalographie (EEG)- oder Elektromyographie (EMG)-Elektroden oder Magnetenzephalographie (MEG)-Sensoren. Die neuronale Aktivität kann auch durch Detektion charakteristischer Bewegungsmuster wie Tremor, Akinese oder epileptische Anfälle mit Hilfe eines Akzelerometers oder Gyroskops oder indirekt durch Messung der Aktivierung des autonomen Nervensystems mittels Messung des Hautleitwiderstands ermittelt werden. Es können auch Befindlichkeitswerte, die vom Patienten in portable Geräte, z. B. Smartphones, eingegeben werden, zur Kontrolle des Stimulationserfolgs verwandt werden.

Alternativ, aber weniger bevorzugt können die Sensoren in den Körper des Patienten implantiert sein. Als invasive Sensoren können beispielsweise epikortikale Elektroden, Tiefenhirnelektroden zur Messung von z. B. lokalen Feldpotentialen, sub- oder epidurale Hirnelektroden, subkutane EEG-Elektroden und sub- oder epidurale Rückenmarkselektroden dienen.

Die Steuereinheit 10 verarbeitet die Signale 24, z. B. können die Signale 24 verstärkt und/oder gefiltert werden, und analysiert die verarbeiteten Signale 24. Die Steuereinheit 10 überprüft anhand der in Reaktion auf die Applikation der Reize 22 aufgenommenen Messsignale 23 den Stimulationserfolg.

Mit Hilfe der von der Messeinheit 16 aufgenommenen Messsignale 23 kann ferner überprüft werden, ob die von der Stimulationseinheit 11 applizierten Bursts 36 die Phase der neuronalen Aktivität der stimulierten Neuronen nicht zurücksetzen. Wie oben beschrieben sollen die Bursts 36 die Phase der neuronalen Aktivität der stimulierten Neuronen nicht zurücksetzen. Eine derartige Untersuchung kann vor der eigentlichen therapeutischen Stimulation, mit welcher die krankhaft synchrone und oszillatorische Aktivität der Neuronenpopulation 31 unterdrückt oder desynchronisiert werden soll, vorgenommen werden.

Dazu wird über einen Sensor der Messeinheit 16 ein Signal gemessen, welches die Aktivität der über den j-ten Stimulationskanal stimulierten Subpopulation hinreichend repräsentiert. Man erhält dieses Signal entweder direkt von der Subpopulation über eine nicht-invasive Messung, z. B. über EEG- oder MEG-Elektroden, oder eine invasive Messung, z. B. über implantierte Elektroden, als Oberflächen-EEG oder als lokales Feldpotential über Tiefenelektroden. Das Signal kann auch indirekt über die Messung einer mit der Aktivität der stimulierten Subpopulation korrelierten Größe ermittelt werden. Hierzu eignen sich z. B. EEG-/MEG-/LFP-Signale der neuronalen Aktivität einer mit dieser Subpopulation eng gekoppelten anderen Neuronenpopulation oder zugehörige Elektromyographie-, Akzelerometer- oder Gyroskop-Signale.

Da neuronale Signale typischerweise rhythmische Aktivität in unterschiedlichen Frequenzbändern enthalten, ist es in solchen Fällen vorteilhaft, z. B. mittels Bandpassfilterung oder wavelet-Analyse oder empirical mode decomposition das Signal $x_j(t)$, welches die pathologische oszillatorische Aktivität der vom j-ten Stimulationskanal stimulierten Subpopulation repräsentiert, zu ermitteln.

Ein nur wenig aufwändiges Vorgehen, um eine Phasenrücksetzung durch die Bursts auszuschließen, besteht darin, die gemittelte Reizantwort zu bestimmen. Hierzu wird zu den Zeiten $\tau_1$, $\tau_2$, ..., $\tau_1$ ein Burst mit identischen Reizparametern appliziert. Die Abstände zwischen den einzelnen Bursts $\tau_{k+1}$ - $\tau_k$ sollten hinreichend groß und randomisiert, also nicht konstant sein, um Einschwingvorgänge zu vermeiden (vgl. P. A. Tass: Transmission of stimulus-locked responses in two coupled phase oscillators.

Phys. Rev. E 69, 051909-1-24 (2004)). Typischerweise sollten die Abstände $\tau_{k+1}$ - $\tau_k$ im Bereich von mindestens dem Zehnfachen, besser dem Hundertfachen der mittleren Periode des pathologischen Oszillation liegen. Die über alle 1 Test-Bursts gemittelte Reizantwort wird gemäß folgender Gleichung berechnet:

$$\overline{x}_j(t) = \frac{1}{l}\sum_{k=1}^{l} x_j(\tau_k + t) \tag{1}$$

Sofern die Abstände $\tau_{k+1}$ - $\tau_k$ zwischen den einzelnen Bursts hinreichend groß sind, erhält man im prä-Stimulus-Bereich, d. h. im Bereich vor der Applikation eines jeweiligen Bursts, keine gemittelte Reizantwort (vgl. P. A. Tass: Transmission of stimulus-locked responses in two coupled phase oscillators. Phys. Rev. E 69, 051909-1-24 (2004)). Eine Phasenrücksetzung kann ausgeschlossen werden, wenn keine gemittelte Reizantwort detektiert werden kann, d. h., wenn sich im post-Stimulus-Bereich, d. h. im Bereich für t > 0, wobei t = 0 den Anfangszeitpunkt des jeweiligen Bursts darstellt, keine von Null verschiedene Reizantwort findet. Dies kann durch visuelle Inspektion ermittelt werden. Man kann dies auch von der Vorrichtung 2, insbesondere der Steuereinheit 10, durchführen lassen, indem man die prä-Stimulus-Verteilung von $\overline{x}_j(t)$ oder $|\overline{x}_j(t)|$ betrachtet und einen charakteristischen Schwellwert, z. B. die 99te Perzentile der prä-Stimulus-Verteilung von $|\overline{x}_j(t)|$ oder schlicht deren Maximum bestimmt. Wenn nun z. B. der Betrag der post-Stimulus-Antwort prinzipiell oder für eine vorgegebene Mindestdauer, z. B. 20 ms, diesen charakteristischen Schwellenwert übersteigt, liegt eine von Null verschiedene gemittelte Antwort vor. In diesem Fall kann eine Phasenrücksetzung nicht mehr ausgeschlossen werden. D. h., die Reizintensität müsste so lange vermindert werden, bis die post-Stimulus-Antwort sich nicht wesentlich von einer Nulllinie unterscheidet. Neben dem hier vorgestellten einfachen, aber in der Praxis bewährten Verfahren können auch andere, dem Fachmann bekannte statistische Tests zur Signalanalyse herangezogen werden.

Eine genauere, aber aufwändigere Variante zur Untersuchung, ob die Bursts eine Phasenrücksetzung bewirken, bietet die Analyse der Phase. Hierzu wird die Phase $\psi_j(t)$ von $x_j(t)$ bestimmt. Dies erfolgt mittels Hilbert-Transformation aus dem mittels Bandpassfilterung bzw. empirical mode decomposition bestimmten Signal, welches die pathologische oszillatorische Aktivität repräsentiert. Die empirical mode decomposition ermöglicht im Vergleich zur Bandpassfilterung eine parameterunabhängige Bestimmung physiologisch relevanter Moden in verschiedenen Frequenzbereichen (vgl. N. E. Huang et al.: The empirical mode decomposition and the Hilbert spectrum for nonlinear and non-stationary time series analysis. Proc. R. Soc. A: Math. Phys. Eng. Sci. 454:903-995 (1998)). Die Kombination von empirical mode decomposition mit nachfolgender Hilbert-Transformation wird als Hilbert-Huang-Transformation bezeichnet (vgl. N. E. Huang et al.: A confidence limit for the empirical mode decomposition and Hilbert spectral analysis, Proceedings of the Royal Society of London Series A, 459, 2317-2345 (2003)). Die Phase $\psi_j(t)$ kann auch mittels Wavelet-Analyse bestimmt werden.

Eine Phasenrücksetzung liegt vor, wenn die Phase $\psi_j(t)$ durch einen Burst (mit Burst-Beginn bei t = 0) nach einer bestimmten Zeit auf einen Vorzugswert gesetzt wird. D. h., $\{\psi_j(\tau_k+t)\}_{k=1,...,l}$, die von den 1 Reizantworten gewonnene Verteilung der Werte der Phase $\psi_j(t)$ hat zur Zeit t (relativ zum Burst-Beginn bei t = 0) einen Häufungswert. Dem Fachmann sind unterschiedliche Methoden bekannt, mit denen sich nachweisen lässt, dass eine Verteilung einen Häufungswert (also einen Peak) hat. Eine gebräuchliche Methode ist die Bestimmung des Phasenrücksetzungsindex ρ(t) mittels zirkulärem Mittelwert:

$$\rho(t) = \left| \frac{1}{l} \sum_{k=1}^{l} \exp\left[ i \psi_j(\tau_k + t) \right] \right| \tag{2}$$

Eine Phasenrücksetzung liegt vor, wenn ρ(t) z. B. das Maximum oder die 99te Perzentile der prä-Stimulus-Verteilung von ρ(t) (an einem Zeitpunkt oder innerhalb eines kleinen Zeitfensters von z. B. 20 ms Breite) überschreitet.

In der Praxis hat sich die Analyse mit den gemittelten Antworten $\overline{x}_j(t)$ als ausreichend bewährt.

Die Bursts können dementsprechend auf folgende Weise kalibriert werden:

1. Der erfahrene Anwender hat entsprechende Erfahrungswerte, die es ihm ermöglichen, im Verlauf einer Therapie oder sogar von Anfang an geeignete Stimulationsparameter einzustellen. D. h., eine Kalibration kann sich erübrigen.
2. Die Reize können aber auch kalibriert werden, z. B. indem bei höheren Reizstärken beginnend die Reizstärke, z. B. (typischerweise) die Amplitude (Intensität) der einzelnen Reize des Bursts oder die Anzahl der im Burst enthaltenen einzelnen Reize, so lange vermindert wird, bis der Burst keine Phasenrücksetzung mehr erzeugt. Man kann z. B. mit der Amplitude 5% oder 10 % oder 20 % oder sogar noch weiter unter die für eine Phasenrücksetzung benötigte Schwellenamplitude gehen.

Gemäß einer Ausgestaltung werden basierend auf der Analyse der verarbeiteten Signale 24 die Parameter der Bursts 36 und/oder die Dauer der Stimulation von der Steuereinheit 10 gesteuert. Beispielsweise können die Dauer $T_{stim}$ der Zyklen, die Wiederholfrequenz fburst der Reize 37 in den Bursts 36, die Intensität der Reize 37, die Dauer D der Reize 37, die Dauer P der Pausen zwischen aufeinanderfolgenden Reizen 37, die Auswahl der Stimulationskanäle, über welche die Stimulation erfolgt, sowie eine bedarfsgesteuerte Stimulation, bei der nur bei Vorliegen von gemessenen pathologischen Markern stimuliert wird, gesteuert werden.

Der Erfolg der Stimulation kann insbesondere mittels eines Schwellwertvergleichs überprüft werden. Je nachdem welche Signale zur Ermittlung des Stimulationserfolgs herangezogen werden, ergeben sich unterschiedliche Schwellwertvergleiche. Wird z. B. die krankhafte neuronale Synchronisation über die Sensoren der Messeinheit 16, z. B. EEG-Elektroden, gemessen, reicht erfahrungsgemäß die Absenkung der Synchronisation um z. B. mindestens 20 % im Vergleich zur Situation ohne Stimulation, um einen ausreichenden Stimulationserfolg festzustellen. Gemäß einer Ausgestaltung kann ein nicht ausreichender Stimulationserfolg festgestellt werden, wenn sich die krankhafte neuronale Synchronisation durch die Applikation der Bursts 36 nicht um mindestens einen vorgegebenen Wert verringert. Falls Symptome des Patienten zur Ermittlung des Stimulationserfolgs herangezogen werden, hängt es von der Art der verwandten klinischen Parameter ab, welche Abnahme als klinisch relevante Besserung anzusehen ist. Derartige Abnahmewerte (z. B. im Sinne der sog. minimalen klinisch wahrnehmbaren Verbesserung) sind dem Fachmann bekannt.

Weiterhin können die Vorrichtungen 1 und 2 ein Eingabegerät umfassen, welches mit der Steuereinheit 10 gekoppelt ist und von dem Patienten oder einer den Patienten behandelnden Person, beispielsweise dem behandelnden Arzt, bedient wird. In das Eingabegerät können Bewertungen des Patienten eingegeben werden, die den subjektiv vom Patienten empfundenen Stimulationserfolg wiedergeben. Beispielsweise kann vorgesehen sein, dass ein oder mehrere Stimulationsparameter geändert werden, falls der von dem Patienten empfundene Stimulationserfolg unter eine vorgegebene Schwelle sinkt. Z. B. können in einem solchen Fall die Wiederholfrequenz fburst der Reize 37 in den Bursts 36, die Intensität der Reize 37, die Dauer D der Reize 37 und/oder die Dauer P der Pausen zwischen aufeinanderfolgenden Reizen 37 variiert werden. Insbesondere kann die Variation der Stimulationsparameter solange erfolgen, bis der von dem Patienten empfundene Stimulationserfolg eine weitere vorgegebene Schwelle überschreitet.

Die einzelnen Komponenten der Vorrichtungen 1 und 2, insbesondere die Steuereinheit 10, die Stimulationseinheit 11 und/oder die Messeinheit 16, können baulich voneinander getrennt sein. Die Vorrichtungen 1 und 2 können daher auch als Systeme aufgefasst werden. Zur Durchführung ihrer Aufgaben kann die Steuereinheit 10 einen Prozessor, z. B. einen Mikrocontroller, enthalten. Die hierin beschriebenen Stimulationsverfahren können als Software-Code in einem der Steuereinheit 10 zugeordneten Speicher abgelegt sein.

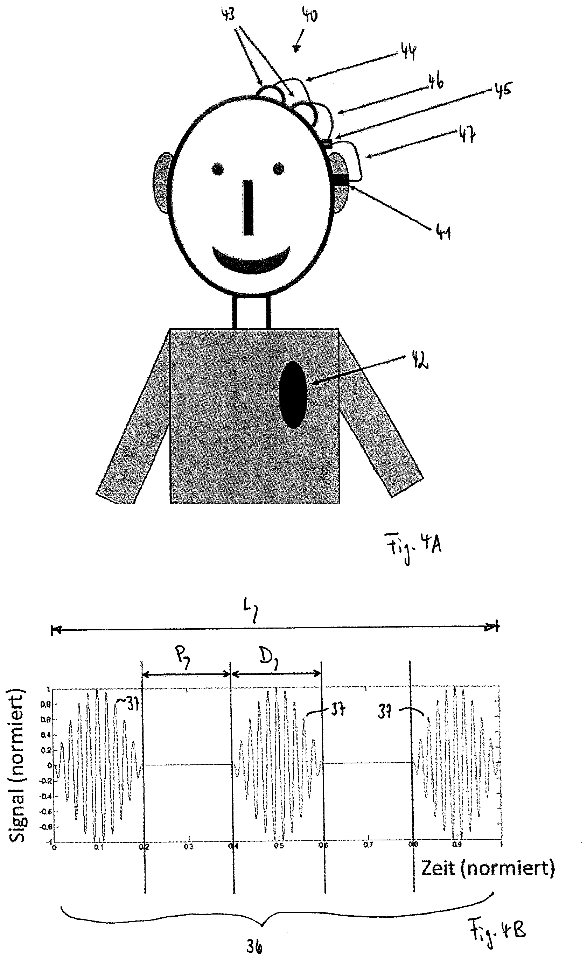

Fig. 4A zeigt schematisch eine Vorrichtung 40 zur nicht-invasiven akustischen Stimulation von Neuronen mit einer krankhaft synchronen und oszillatorischen neuronalen Aktivität gemäß einer Ausführungsform der Erfindung. Akustische Reize werden dem Patienten über Ohr- oder Kopfhörer 41 oder anders ausgestaltete Lautsprecher verabreicht, wobei ein

Ohrhörer ein im Ohrkanal platzierter Lautsprecher ist. Die hierzu verwendeten Steuersignale werden von einer Steuereinheit 42 generiert. Nicht-invasiv fixierte EEG-Elektroden 43, die über ein Kabel 44 verbunden sind, dienen der "closed loop"-Stimulation. Die entsprechende Verrechnung wird in einem kleinen Bauteil 45, das vorzugsweise einen Messverstärker enthält und über Kabel 46, 47 mit den EEG-Elektroden 43 bzw. dem Ohr- oder Kopfhörer 41 verbunden ist, und/oder in der eigentlichen, die Batterie bzw. den Akku beherbergenden Steuereinheit 42 durchgeführt. Die Steuereinheit 42 und das Bauteil 45 sind in der in Fig. 4 dargestellten Ausführungsform telemetrisch miteinander verbunden; in diesem Fall enthält Bauteil 45 (oder ein mit ihm über Kabel verbundenes Bauteil) ebenso eine Batterie bzw. einen Akku. Alternativ können die Steuereinheit 42 und das Bauteil 45 auch über Kabel miteinander verbunden sein, so dass das Bauteil 45 über die Stromversorgung von der Steuereinheit 42 gespeist wird.

Fig. 4B zeigt als Beispiel einen Burst 36, der mit der Vorrichtung 40 appliziert werden kann. Der Burst 36 enthält drei aufeinanderfolgende als einzelne Töne ausgestaltete akustische Reize 37. In der Darstellung von Fig. 4B sind sowohl das Stimulationssignal als auch die Zeit normiert. Dazu sind das Maximum des Stimulationssignals und die Gesamtdauer L des Bursts 36 jeweils auf 1 gesetzt. Jeder Reiz 37 besteht aus einer hochfrequenten Sinusschwingung mit einer niederfrequenteren Einhüllenden. Die Einhüllende kann, wie in Fig. 4B gezeigt ist, eine Sinus- oder Cosinus-Halbschwingung sein. Sie kann aber auch ein Hanning-Fenster, z. B. mit $\cos^2$, oder ein Hamming-Fenster oder eine andere Fensterfunktion sein, bei der sich um die Mittenfrequenz ein schmales Spektrum findet, während außerhalb der Mitte starke Dämpfungen erzielt werden. Durch die Dämpfung zu Beginn und am Ende des jeweiligen Reizes 37 werden Geräusche, die für den Patienten wie ein Klicken oder ein dumpfer Schlag klingen, vermieden.

Die Frequenz der hochfrequente Sinusschwingung kann im Bereich von 200 Hz bis 13 kHz liegen. Die Dauer D eines Reizes 37 kann beispielsweise im Bereich von 10 ms bis 60 ms und die Pause P zwischen aufeinanderfolgenden Reizen 37 innerhalb eines Bursts 36 kann im Bereich von 10 ms bis 50 ms liegen.

Der in Fig. 4B gezeigte Burst 36 ist für die Stimulation über einen Stimulationskanal ausgelegt. Wie weiter unten detailliert beschrieben wird, ist bei der akustischen Stimulation jeder Stimulationskanal einem jeweiligen Frequenzbereich zugeordnet, aus dem die Töne, die als akustische Reize in dem jeweiligen Stimulationskanal appliziert werden, ausgewählt werden. Demnach liegt die in Fig. 4A gezeigte hochfrequente Sinusschwingung der Reize 37 in dem Frequenzbereich des gewünschten Stimulationskanals. Die in anderen Stimulationskanälen applizierten Reize 37 weisen Sinusschwingungen mit anderen Frequenzen auf.

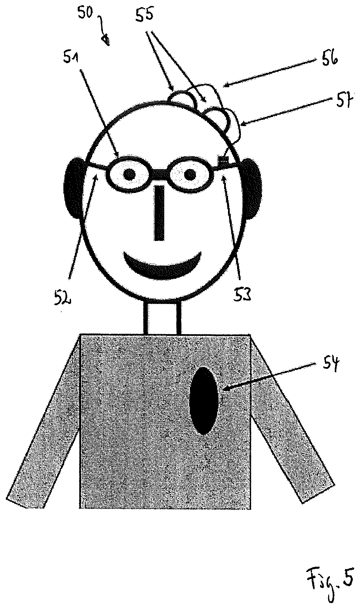

Fig. 5 zeigt schematisch eine Vorrichtung 50 zur nicht-invasiven visuellen Stimulation von Neuronen mit einer krankhaft synchronen und oszillatorischen neuronalen Aktivität gemäß einer Ausführungsform der Erfindung. Bei dieser Ausgestaltung trägt der Patient eine Stimulationsbrille 51, die z. B. über Bügel 52 am Kopf des Patienten befestigt ist. Ein Bauteil 53 enthält eine Verrechnungs- und Telemetrieeinheit. Letztere dient der Verbindung mit der eigentlichen, die Batterie bzw. den Akku beherbergenden Steuereinheit 54. Das Bauteil 53 und die Steuereinheit 54 sind telemetrisch miteinander verbunden; in diesem Fall enthält Bauteil 53 (oder ein mit ihm über Kabel verbundenes Bauteil) ebenso eine Batterie bzw. einen Akku. Alternativ können das Bauteil 53 und die Steuereinheit 54 auch über Kabel miteinander verbunden sein. Nicht-invasiv fixierte EEG-Elektroden 55 dienen der "closed loop"-Stimulation. Die EEG-Elektroden 55 sind über Kabel 56, 57 mit dem Bauteil 53 verbunden.

Den von der Stimulationsbrille 51 erzeugten visuellen Reizen kann eine Leuchtstärken- bzw. Helligkeitsvariation (bzw. Variation der Lichtintensität oder Lichtstärke) zugrunde liegen, beispielsweise können sie als Pulse oder als Sequenzen von Pulsen mit variierter Leuchtstärke bzw. Helligkeit appliziert werden. Die visuellen Reize können je nach Ausgestaltung als Leuchtstärkenmodulation natürlicher visueller Reize, z. B. mittels einer homogenen oder segmentierten Transmissionsbrille, bei der spannungsabhängig die Transmission geregelt werden kann, als zusätzlich zu einem natürlichen visuellen Reiz auftretender, modulierter visueller Reiz, z. B. mittels einer partiell durchsichtigen Lichtbrille, oder als künstlicher visueller Helligkeitsreiz, z. B. mittels einer undurchsichtigen Lichtbrille, verabreicht werden. Die Stimulationsbrille 51 ist vorzugsweise in unterschiedliche Segmente unterteilt, deren Leuchtstärke bzw. Transmission bzw. Helligkeit getrennt gesteuert werden kann, um verschiedene Stellen der Retina unabhängig voneinander stimulieren zu können.

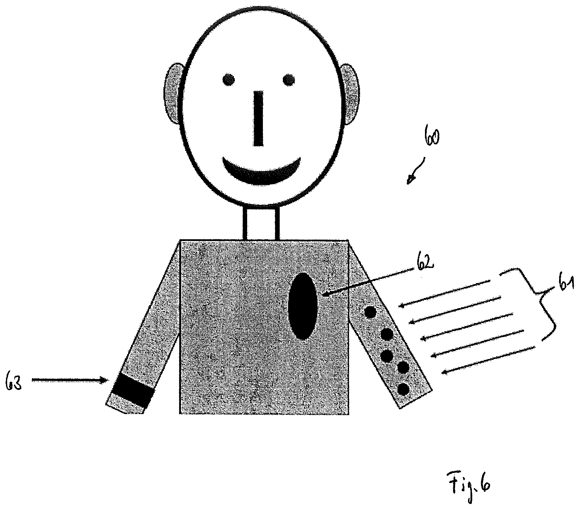

Fig. 6 zeigt schematisch eine Vorrichtung 60 zur nicht-invasiven taktilen, vibratorischen, thermischen, transkutanen elektrischen und/oder transkutanen magnetischen Stimulation und/oder Ultraschall-Stimulation von Neuronen mit einer krankhaft synchronen und oszillatorischen neuronalen Aktivität gemäß einer Ausführungsform der Erfindung. Die Vorrichtung 60 umfasst eine Stimulationseinheit 61, eine die Stimulationseinheit 61 ansteuernde Steuereinheit 62 und ein Akzelerometer (Beschleunigungsmesser) 63 zum Aufnehmen von Messsignalen. Die Stimulationseinheit 61 und das Akzelerometer 63 können mit der Steuereinheit 62 telemetrisch oder über Kabel verbunden sein.

Die Stimulationseinheit 61 umfasst eine Mehrzahl von Stimulationselementen zur Erzeugung von taktilen, vibratorischen, thermischen, transkutanen elektrischen und/oder transkutanen magnetischen Reizen und/oder Ultraschall-Reizen. Die Stimulationselemente sind derart ausgestaltet, dass sie auf die Haut des Patienten aufgesetzt werden können. Je nach Erkrankung bzw. betroffenen Körperpartien werden die Stimulationselemente in einer geeigneten Anordnung auf der Haut des Patienten befestigt, beispielsweise am Arm, am Bein, an der Hand und/oder am Fuß des Patienten. Die Mehrzahl von Stimulationselementen ermöglicht es, unterschiedliche rezeptive Bereiche der Haut über die einzelnen Stimulationselemente zeitlich und räumlich koordiniert zu stimulieren.

Stimulationselemente zur Erzeugung von taktilen und/oder vibratorischen Reizen 37 sind beispielsweise Vibrationsaktoren, die mit einer Frequenz im Bereich von 1 bis 300 Hz und insbesondere 1 bis 60 Hz und vorzugsweise 100 bis 300 Hz, dem Resonanzbereich der in der Haut gelegenen Vibrationsrezeptoren, in die Haut des Patienten eindrücken und dadurch die gewünschten Reize 37 erzeugen. Die Dauer D eines Reizes 37 kann beispielsweise im Bereich von 20 ms bis 80 ms und die Pause P zwischen aufeinanderfolgenden Reizen 37 innerhalb eines Bursts 36 kann im Bereich von 15 ms bis 40 ms liegen.

Stimulationselemente zur Erzeugung thermischer Reize können beispielsweise Laser oder anders ausgestaltete Elemente zur Erzeugung von Wärme, insbesondere Wärmestrahlung, sein. Zur Erzeugung von transkutanen elektrischen Reizen werden typischerweise Elektroden auf der Haut des Patienten befestigt. Transkutane magnetische Reize können durch entsprechende Stimulationselemente zur Erzeugung magnetischer Reize, insbesondere stromdurchflossene Spulen, erzeugt werden. Ultraschall-Reize werden durch Stimulationselemente zur Erzeugung von UltraschallWellen erzeugt.

Bei der Applikation akustischer oder visueller Reize werden diese über mindestens ein Ohr bzw. mindestens ein Auge des Patienten aufgenommen. Die taktilen, vibratorischen, thermischen, transkutanen elektrischen und/oder transkutanen magnetischen Reize und/oder Ultraschall-Reize können von in oder unter der Haut gelegenen Rezeptoren aufgenommen und an das Nervensystem weitergeleitet werden. Zu diesen Rezeptoren zählen beispielsweise Merkel-Zellen, Ruffini-Körperchen, Meissner-Körperchen und Haarfollikelrezeptoren, die insbesondere als Rezeptoren für die taktilen Reize wirken. Die vibratorischen Reize zielen vorwiegend auf die Tiefensensibilität ab. Die vibratorischen Reize können von in der Haut, den Muskeln, dem Subkutangewebe und/oder den Sehnen des Patienten gelegenen Rezeptoren aufgenommen werden. Als Rezeptoren für die vibratorischen Reize seien beispielhaft die Vater-Pacini-Körperchen genannt, die Vibrationsempfindungen und Beschleunigungen vermitteln. Die thermischen Reize werden von den Thermorezeptoren der Haut aufgenommen. Dies sind Warmrezeptoren (auch Wärmerezeptoren, Warmsensoren oder Wärmesensoren genannt) und Kaltsensoren (auch Kältesensoren, Kaltrezeptoren oder Kälterezeptoren genannt). In der Haut des Menschen liegen die Kaltsensoren mehr oberflächlich, die Warmrezeptoren etwas tiefer. Die transkutanen elektrischen und transkutanen magnetischen Reize sowie die Ultraschall-Reize wirken nicht spezifisch auf nur eine Gruppe von in oder unter der Haut gelegenen Rezeptoren und können darüber hinaus auch direkt Nervenfasern reizen.

Die gezielte Stimulation bestimmter Bereiche des Gehirns oder Rückenmarks wird durch die tonotope bzw. somatotope Zuordnung von Körperregionen zu diesen Bereichen ermöglicht. Beispielsweise werden akustische Reize im Innenohr in Nervenimpulse umgesetzt und über den Hörnerv zu dem auditorischen Cortex weitergeleitet. Durch die tonotope Anordnung des auditorischen Cortex wird bei der akustischen Stimulation des Innenohres mit einer bestimmten Frequenz ein bestimmter Teil des auditorischen Cortex aktiviert.

Bei der visuellen Stimulation werden unterschiedliche Stellen im Gesichtsfeld über die Linse des Auges auf unterschiedliche Stellen der Retina abgebildet. Die unterschiedlichen Stellen der Retina sind wiederum über den Sehnerv mit unterschiedlichen Neuronen im Gehirn verbunden. Folglich können mit den an unterschiedlichen räumlichen Orten applizierten Reizen jeweils unterschiedliche Neuronen stimuliert werden.

Aufgrund der somatotopen Gliederung der Nervenleitungsbahnen und zugehörigen Hirngebiete werden des Weiteren durch taktile, vibratorische, thermische, transkutane elektrische und/oder transkutane magnetische Reize und/oder Ultraschall-Reize, die an unterschiedlichen Stellen der Haut appliziert werden, unterschiedliche Neuronen stimuliert. Bei diesen Stimulationsformen können die Stimulationselemente beispielsweise am Fuß, Unterschenkel und Oberschenkel oder aber an der Hand, dem Unterarm und Oberarm des Patienten angebracht werden, um dadurch bestimmte Neuronen stimulieren zu können.

Bei der gustatorischen Reizung werden unterschiedliche Bereich der Zunge mit den entsprechenden Geschmacksqualitäten - süß, sauer, salzig, bitter und umami (japanisch für pikantes, würziges, bouillonartiges Aroma) - gereizt. Es ist aber auch möglich, die Zunge elektrisch zu reizen. In diesem Fall reizt man primär die Schleimhaut, welche im Homunculus (Repräsentation der Oberfläche des Menschen im sensomotorischen Cortex) eine erheblich große Repräsentation, d. h. ein erheblich großes zugeordnetes Areal, aktiviert. Aufgrund der somatotopen Gliederung der Nervenleitungsbahnen und zugehörigen Hirngebiete werden durch gustatorische Reize, die an unterschiedlichen Stellen der Zunge appliziert werden, unterschiedliche Neuronen stimuliert.

Ganz allgemein und nicht nur bezogen auf die hier beschriebenen Ausführungsbeispiele gilt Folgendes. Bei der akustischen Stimulation ist jeder Stimulationskanal einem jeweiligen unterschiedlichen Frequenzbereich zugeordnet, aus dem die Töne, die als akustische Reize in dem jeweiligen Stimulationskanal appliziert werden, ausgewählt werden. Bei der visuellen Stimulation werden die Stimulationskanäle durch unterschiedliche Stellen oder Bereiche im Gesichtsfeld des Patienten bestimmt. Die in einem jeweiligen Stimulationskanal erzeugten visuellen Reize werden an einer jeweiligen Stelle bzw. in einem jeweiligen Bereich des Gesichtsfelds erzeugt. Die Stimulationskanäle der taktilen, vibratorischen, thermischen, transkutanen elektrischen und/oder transkutanen magnetischen Reize und/oder Ultraschall-Reize werden durch die Stellen der Haut, welche mit den jeweiligen Stimulationselementen stimuliert werden, bestimmt. Folglich ist jeder Stimulationskanal einer jeweiligen Stelle bzw. einem jeweiligen Bereich der Haut zugeordnet.

Die Stimulationskanäle der gustatorischen Reize werden durch die Stellen der Zunge, welche mit den entsprechenden Geschmacksqualitäten oder elektrischen Reizen stimuliert werden, bestimmt. Bei einer olfaktorischen Stimulation verwendet man psychophysisch hinreichend disjunkte Geruchsreize, durch welche die Stimulationskanäle festgelegt würden. Die psychophysisch hinreichend disjunkten Geruchsreize könnten z. B. personalisiert, d. h. an den individuellen Patienten angepasst sein.

Bei der transkraniellen elektrischen und transkraniellen magnetischen Stimulation werden Elektroden bzw. Magnetfeldgeneratoren, insbesondere stromdurchflossene Spulen, am Körper, insbesondere am Kopf, des Patienten befestigt. Durch die Elektroden und Magnetfeldgeneratoren können Ströme bzw. Magnetfelder im Gehirn und/oder Rückenmark des Patienten erzeugt werden. Je nach Anbringungsort der Elektroden bzw. Magnetfeldgeneratoren können unterschiedliche Zielgebiete im Gehirn und/oder Rückenmark stimuliert werden. Die Stimulationskanäle werden folglich durch die Stellen am Körper des Patienten, an denen die Elektroden bzw. Magnetfeldgeneratoren angebracht sind, bestimmt.

Die vorstehend beschriebene Stimulationseinheit kann demnach unterschiedliche Bereiche des Gehirns oder Rückenmarks über verschiedene Stimulationskanäle separat stimulieren, indem die applizierten Reize über Nervenleitungen an unterschiedliche Zielgebiete, die im Gehirn und/oder Rückenmark liegen, weitergeleitet werden. Die Zielgebiete können während der Stimulation mit eventuell unterschiedlichen und/oder zeitversetzten Reizen stimuliert werden.

## Patentansprüche

1. Vorrichtung (2) zur Stimulation von Neuronen, umfassend

   - eine nicht-invasive Stimulationseinheit (11) zur Erzeugung von Reizen (22) in einer Mehrzahl von Stimulationskanälen (12-15), wobei die Stimulationseinheit (11) derart ausgelegt ist, dass die Reize (22) eine Neuronenpopulation (31) im Gehirn und/oder Rückenmark (30) eines Patienten über die Stimulationskanäle (12-15) an jeweils unterschiedlichen Stellen stimulieren,
   - eine Messeinheit (16) zum Aufnehmen von Messsignalen (23), die eine neuronale Aktivität der mit den Reizen (22) stimulierten Neuronenpopulation (31) wiedergeben, und
   - eine Steuereinheit (10), welche die Stimulationseinheit (11) derart ansteuert, dass die Stimulationseinheit (11) in jedem der Stimulationskanäle (12-15) repetitiv Bursts (36), die jeweils mehrere Reize (37) umfassen, erzeugt, wobei die in unterschiedlichen Stimulationskanälen (12-15) erzeugten Bursts (36) gegeneinander zeitversetzt sind,

   **dadurch gekennzeichnet, dass**

   - die Bursts (36) derart ausgelegt sind, dass sie die Phase der neuronalen Aktivität der jeweils stimulierten Neuronen nicht zurücksetzen,
   - die Steuereinheit (10) anhand der Messsignale (23) prüft, ob von der Stimulationseinheit (11) erzeugte Bursts (36) die Phase der neuronalen Aktivität der jeweils stimulierten Neuronen nicht zurücksetzen,
   - die Steuereinheit (10), nachdem festgestellt wurde, dass die Bursts (36) die Phase der neuronalen Aktivität der jeweils stimulierten Neuronen zurücksetzen, die Stimulationseinheit (11) derart ansteuert, dass die von der Stimulationseinheit (11) nachfolgend erzeugten Bursts (36) eine verringerte Reizstärke aufweisen, und
   - die Verringerung der Reizstärke der von der Stimulationseinheit (11) erzeugten Bursts (36) solange fortgesetzt wird, bis die Steuereinheit (10) feststellt, dass die Bursts (36) die Phase der neuronalen Aktivität der jeweils stimulierten Neuronen nicht zurücksetzen.

2. Vorrichtung (2) nach Anspruch 1, wobei die Reize (22) akustische, visuelle, taktile, vibratorische, thermische, olfaktorische, gustatorische, transkutane elektrische, transkutane magnetische, transkranielle elektrische und/oder transkranielle magnetische Reize und/oder Ultraschall-Reize sind.

3. Vorrichtung (2) nach Anspruch 1 oder 2, wobei jeweils zwei zeitlich aufeinanderfolgende und in unterschiedlichen Stimulationskanälen (12-15) erzeugte Bursts (36) gegeneinander um $T_{stim}/S$ zeitversetzt sind, wobei $T_{stim} = 1/f_{stim}$ gilt und $f_{stim}$ eine Frequenz im Bereich von 1 bis 30 Hz ist und S die Anzahl der Stimulationskanäle (12-15) ist.

4. Vorrichtung (2) nach Anspruch 1 oder 2, wobei die Stimulationseinheit (11) die Bursts (36) in einem Zeitraster, das aus aufeinanderfolgenden Zyklen besteht, erzeugt und die Zyklen jeweils eine Dauer $T_{stim}$ aufweisen, wobei $T_{stim} = 1/f_{stim}$ gilt und $f_{stim}$ eine Frequenz im Bereich von 1 bis 30 Hz ist.

**5.** Vorrichtung (2) nach Anspruch 4, wobei die Stimulationseinheit (11) in einem der Zyklen in jedem Stimulationskanal (12-15) genau einen Burst (36) erzeugt.

**6.** Vorrichtung (2) nach Anspruch 4 oder 5, wobei jeder der Zyklen in S insbesondere gleich lange Subzyklen unterteilt ist und die Stimulationseinheit (11) in jedem Subzyklus nicht mehr als einen Burst (36) erzeugt, wobei S die Anzahl der Stimulationskanäle (12-15) ist.

**7.** Vorrichtung (2) nach einem der Ansprüche 4 bis 6, wobei die Stimulationseinheit (11) während n aufeinanderfolgenden Zyklen Bursts (36) erzeugt und während der darauffolgenden m Zyklen keine Reize erzeugt und dieses Muster periodisch fortgesetzt wird, wobei n und m nicht-negative ganze Zahlen sind.

**8.** Vorrichtung (2) nach einem der Ansprüche 4 bis 7, wobei die Bursts (36) dazu ausgelegt sind, bei einer Verabreichung an den Patienten über die Mehrzahl von Stimulationskanälen (12-15) eine krankhaft synchrone und oszillatorische Aktivität der Neuronenpopulation (31) zu unterdrücken.

**9.** Vorrichtung (2) nach Anspruch 8, wobei die Dauer $T_{stim}$ eines Zyklus im Wesentlichen der mittleren Periode der pathologischen Oszillation der Neuronenpopulation (31) entspricht.

**10.** Vorrichtung (2) nach einem der vorhergehenden Ansprüche, wobei die Reizstärke eines Bursts (36) verringert wird, indem die Amplitude und/oder die Anzahl der in dem Burst (36) enthaltenen Reize (37) verringert wird.

**11.** Vorrichtung (2) nach einem der vorhergehenden Ansprüche, wobei für die Prüfung, ob die von der Stimulationseinheit (11) generierten Bursts (36) die Phase der neuronalen Aktivität der stimulierten Neuronen nicht zurücksetzen, mehrere identische Bursts (36) nacheinander generiert werden und die Steuereinheit (10) die als Antwort auf die Applikation eines jeden der mehreren Bursts (36) von der Messeinheit (16) aufgenommene neuronale Aktivität bestimmt und feststellt, dass die Bursts (36) die Phase der neuronalen Aktivität der stimulierten Neuronen nicht zurücksetzen, wenn die über alle Antwortsignale gemittelte neuronale Aktivität nicht von Null verschieden ist.

**12.** Vorrichtung (2) nach einem der Ansprüche 1 bis 10, wobei für die Prüfung, ob die von der Stimulationseinheit (11) generierten Bursts (36) die Phase der neuronalen Aktivität der stimulierten Neuronen nicht zurücksetzen, mehrere identische Bursts (36) nacheinander generiert werden und die Steuereinheit (10) die Phase der als Antwort auf die Applikation eines jeden der mehreren Bursts (36) von der Messeinheit (16) aufgenommenen neuronalen Aktivität bestimmt und feststellt, dass die Bursts (36) die Phase der neuronalen Aktivität der stimulierten Neuronen nicht zurücksetzen, wenn die Verteilung der Phasen keinen Häufungswert aufweist.

**13.** Verfahren zur Stimulation von Neuronen (31), bei welchem

- eine nicht-invasive Stimulationseinheit (11) Reize (22) in einer Mehrzahl von Stimulationskanälen (12-15) erzeugt, wobei
- die Reize (22) eine Neuronenpopulation (31) im Gehirn und/oder Rückenmark (30) eines Patienten über die Stimulationskanäle (12-15) an jeweils unterschiedlichen Stellen stimulieren,
- Messsignale (23) aufgenommen werden, die eine neuronale Aktivität der mit den Reizen (22) stimulierten Neuronenpopulation (31) wiedergeben,
- die Stimulationseinheit (11) in jedem der Stimulationskanäle (12-15) repetitiv Bursts (36), die jeweils mehrere Reize (37) umfassen, erzeugt,
- die in unterschiedlichen Stimulationskanälen (12-15) erzeugten Bursts (36) gegeneinander zeitversetzt sind,
- die Bursts (36) die Phase der neuronalen Aktivität der jeweils stimulierten Neuronen nicht zurücksetzen,
- anhand der Messsignale (23) geprüft wird, ob von der Stimulationseinheit (11) erzeugte Bursts (36) die Phase der neuronalen Aktivität der jeweils stimulierten Neuronen nicht zurücksetzen,
- nachdem festgestellt wurde, dass die Bursts (36) die Phase der neuronalen Aktivität der jeweils stimulierten Neuronen zurücksetzen, die Stimulationseinheit (11) nachfolgend Bursts (36) erzeugt, die eine verringerte Reizstärke aufweisen, und
- die Verringerung der Reizstärke der von der Stimulationseinheit (11) erzeugten Bursts (36) solange fortgesetzt wird, bis festgestellt wird, dass die Bursts (36) die Phase der neuronalen Aktivität der jeweils stimulierten Neuronen nicht zurücksetzen.

**14.** Software zum Ausführen in einem Datenverarbeitungssystem, wobei die Software

- Steuersignale zum Ansteuern einer nicht-invasiven Stimulationseinheit (11) erzeugt, wobei die Stimulationseinheit (11) Reize (22) in einer Mehrzahl von Stimulationskanälen (12-15) erzeugt und die Reize (22) eine Neuronenpopulation (31) im Gehirn und/oder Rückenmark (30) eines Patienten über die Stimulationskanäle (12-15) an jeweils unterschiedlichen Stellen stimulieren und wobei Messsignale (23) aufgenommen werden, die eine neuronale Aktivität der mit den Reizen (22) stimulierten Neuronenpopulation (31) wiedergeben, wobei die Steuersignale die Stimulationseinheit (11) derart ansteuern, dass

- die Stimulationseinheit (11) in jedem der Stimulationskanäle (12-15) repetitiv Bursts (36), die jeweils mehrere Reize (37) umfassen, erzeugt,

- die in unterschiedlichen Stimulationskanälen (12-15) erzeugten Bursts (36) gegeneinander zeitversetzt sind,

- die Bursts (36) die Phase der neuronalen Aktivität der jeweils stimulierten Neuronen nicht zurücksetzen,

- anhand der Messsignale (23) geprüft wird, ob von der Stimulationseinheit (11) erzeugte Bursts (36) die Phase der neuronalen Aktivität der jeweils stimulierten Neuronen nicht zurücksetzen,

- nachdem festgestellt wurde, dass die Bursts (36) die Phase der neuronalen Aktivität der jeweils stimulierten Neuronen zurücksetzen, die Stimulationseinheit (11) nachfolgend Bursts (36) erzeugt, die eine verringerte Reizstärke aufweisen, und

- die Verringerung der Reizstärke der von der Stimulationseinheit (11) erzeugten Bursts (36) solange fortgesetzt wird, bis festgestellt wird, dass die Bursts (36) die Phase der neuronalen Aktivität der jeweils stimulierten Neuronen nicht zurücksetzen.

**Claims**

1. Apparatus (2) for stimulating neurons, comprising

- a non-invasive stimulation unit (11) for the generation of stimuli (22) in a plurality of stimulation channels (12-15), wherein the stimulation unit (11) is configured in such a way that the stimuli (22) stimulate a neuron population (31) in the brain and/or the spinal cord (30) of a patient via the stimulation channels (12-15) at respectively different points;

- a measurement unit (16) for the recording of measurement signals (23) that represent a neuronal activity of the neuron population (31) stimulated with the stimuli (22); and

- a control unit (10) which controls the stimulation unit (11) in such a way that the stimulation unit (11) generates repetitive bursts (36) that each comprise a plurality of stimuli (37) in each of the stimulation channels (12-15), wherein the bursts (36) generated in different stimulation channels (12-15) are delayed in time with respect to one another,

**characterised in that**

- the bursts (36) are configured in such a way that they do not reset the phase of the neuronal activity of the respective stimulated neurons,

- the control unit (10) by means of the measurement signals (23) checks whether the bursts (36) generated by the stimulation unit (11) do not reset the phase of the neuronal activity of the respectively stimulated neurons,

- following a determination that the bursts (36) reset the phase of the neuronal activity of the respectively stimulated neurons, the control unit (10) controls the stimulation unit (11) in such a way that the bursts (36) subsequently generated by the stimulation unit (11) have a reduced stimulation strength, and

- the reduction of the stimulation strength of the bursts (36) generated by the stimulation unit (11) is continued for so long until the control unit (10) recognizes that the bursts (36) do not reset the phase of the neuronal activity of the respectively stimulated neurons.

2. Apparatus (2) in accordance with claim 1, wherein the stimuli (22) are acoustic stimuli, visual stimuli, tactile stimuli, vibratory stimuli, olfactory stimuli, gustatory stimuli, transcutaneous electric stimuli, transcutaneous magnetic stimuli, transcranial electrical stimuli and/or transcranial magnetic stimuli and/or ultrasound stimuli.

3. Apparatus (2) in accordance with claim 1 or claim 2, wherein two respective bursts (36) following one another in time and generated in different stimulation channels (12-15) are delayed in time with respect to one another by $T_{stim}/S$; wherein $T_{stim} = 1/f_{stim}$ is true and $f_{stim}$ is a frequency in the range of 1 to 30 Hz and S is the number of stimulation channels (12-15).

4. Apparatus (2) in accordance with claim 1 or claim 2, wherein the stimulation unit (11) generates the bursts (36) in a time pattern that is made up of consecutive cycles and the cycles respectively have a duration $T_{stim}$, wherein $T_{stim}$ = $1/f_{stim}$ is true and $f_{stim}$ is a frequency in the range of 1 to 30 Hz.

5. Apparatus (2) in accordance with claim 4, wherein the stimulation unit (11) generates exactly one burst (36) in one of the cycles in each stimulation channel (12-15).

6. Apparatus (2) in accordance with claim 4 or claim 5, wherein each of the cycles is divided into S sub-cycles, in particular into S sub-cycles of equal length and the stimulation unit (11) generates no more than one burst (36) in each sub-cycle, wherein S is the number of stimulation channels (12-15).

7. Apparatus (2) in accordance with any one of the claims 4 to 6, wherein the stimulation unit (11) generates bursts (36) during n successive cycles and during the m cycles following these generates no stimuli and this pattern is periodically continued, wherein n and m are non-negative whole numbers.

8. Apparatus (2) in accordance with any one of the claims 4 to 7, wherein the bursts (36) are configured to suppress a pathological synchronous and oscillatory activity of the neuron population (31) on an administration to the patient via the plurality of stimulation channels (12-15).

9. Apparatus (2) in accordance with claim 8, wherein the duration $T_{stim}$ of a cycle substantially corresponds to the mean period of the pathological oscillation of the neuron population (31).

10. Apparatus (2) in accordance with any one of the preceding claims, wherein the stimulation strength of a burst (36) is reduced in that the amplitude and/or the number of stimuli (37) included in the bursts (36) are reduced.

11. Apparatus (2) in accordance with any one of the preceding claims, wherein for the check of whether the bursts (36) generated by the stimulation unit (11) do not reset the phase of the neuronal activity of the stimulated neurons, a plurality of identical bursts (36) are generated one after the other, and the control unit (10) determines the neuronal activity recorded by the measurement unit (16) as a response to the application of each of the plurality of bursts (36) and recognizes that the bursts (36) do not reset the phase of the neuronal activity of the stimulated neurons, when the neuronal activity averaged over all response signals is not different from zero.

12. Apparatus (2) in accordance with any one of the claims 1 to 10, wherein for the check of whether the bursts (36) generated by the stimulation unit (11) do not reset the phase of a neuronal activity of the stimulated neurons, a plurality of identical bursts (36) are generated one after the other, and the control unit (10) determines the phase of the neuronal activity recorded by the measurement unit (16) as a response to the application of each of the plurality of bursts (36) and recognizes that the bursts (36) do not reset the phase of the neuronal activity of the stimulated neurons when the distribution of the phases has no accumulation point.

13. Method for the stimulation of neurons (31) in which

- a non-invasive stimulation unit (11) generates stimuli (22) in a plurality of stimulation channels (12-15), wherein
- the stimuli (22) stimulate a neuron population (31) in the brain and/or the spinal cord (30) of a patient via the stimulation channels (12-15) at respectively different points,
- measurement signals (23) that represent a neuronal activity of the neuron population (31) stimulated with the stimuli (22) are recorded,
- the stimulation unit (11) generates repetitive bursts (36) in each of the stimulation channels (12-15) that each comprise a plurality of stimuli (37), wherein the bursts (36) generated in different stimulation channels (12-15) are delayed in time with respect to one another,
- the bursts (36) do not reset the phase of a neuronal activity of the respectively stimulated neurons,
- the control unit (10) by means of the measurement signals (23) checks whether the bursts (36) generated by the stimulation unit (11) do not reset the phase of the neuronal activity of the respectively stimulated neurons,
- following a determination that the bursts (36) reset the phase of the neuronal activity of the respectively stimulated neurons, the control unit (10) controls the stimulation unit (11) in such a way that the bursts (36) subsequently generated by the stimulation unit (11) have a reduced stimulation strength, and
- the reduction of the stimulation strength of the bursts (36) generated by the stimulation unit (11) is continued for so long until the control unit (10) recognizes that the bursts (36) do not reset the phase of the neuronal activity of the respectively stimulated neurons.

**14.** Software for execution in a data processing system, wherein the software

- generates control signals for controlling a non-invasive stimulation unit (11), wherein the stimulation unit (11) generates stimuli (22) in a plurality of stimulation channels (12-15) and the stimuli (22) stimulate a neuron population (31) in the brain and/or the spinal cord (30) of a patient via the stimulation channels (12-15) at respectively different points and wherein measurement signals (23) that represent a neuronal activity of the neuron population (31) stimulated with the stimuli (22) are recorded, wherein the control signals control the stimulation unit (11) in such a way that
- the stimulation unit (11) generates repetitive bursts (36) that each comprise a plurality of stimuli (37) in each of the stimulation channels (12-15),
- the bursts (36) generated in different stimulation channels (12-15) are delayed in time with respect to one another,
- the bursts (36) do not reset the phase of a neuronal activity of the respectively stimulated neurons,
- the control unit (10) by means of the measurement signals (23) checks whether the bursts (36) generated by the stimulation unit (11) do not reset the phase of the neuronal activity of the respectively stimulated neurons,
- following a determination that the bursts (36) reset the phase of the neuronal activity of the respectively stimulated neurons, the control unit (10) controls the stimulation unit (11) in such a way that the bursts (36) subsequently generated by the stimulation unit (11) have a reduced stimulation strength, and
- the reduction of the stimulation strength of the bursts (36) generated by the stimulation unit (11) is continued for so long until the control unit (10) recognizes that the bursts (36) do not reset the phase of the neuronal activity of the respectively stimulated neurons.

**Revendications**

**1.** Un dispositif (2) de stimulation des neurones comprenant

- une unité de stimulation non-invasive (11) pour générer des stimuli (22) dans une pluralité de canaux de stimulation (12 - 15), l'unité de stimulation (11) étant conçue de manière que les stimuli (22) stimulent une population neuronale (31) dans le cerveau et/ou dans la moelle épinière (30) d'un patient via les canaux de stimulation (12 - 15) à différents endroits,
- une unité de mesure (16) pour enregistrer des signaux de mesure (23) représentant une activité neuronale de la population neuronale (31) stimulée par les stimuli (22), et
- une unité de commande (10) qui commande l'unité de stimulation (11) de manière que l'unité de stimulation (11) génère de manière répétitive des rafales (36) dans chacun des canaux de stimulation (12 - 15), lesquelles rafales comprennent chacune une pluralité de stimuli (37), sachant que les rafales (36) générées dans différents canaux de stimulation (12 - 15) sont décalées dans le temps les unes par rapport aux autres,

**caractérisé en ce que**

- les rafales (36) sont adaptées de manière qu'elles ne réinitialisent pas la phase de l'activité neuronale des neurones respectivement stimulés,
- l'unité de commande (10) vérifie à l'aide des signaux de mesure (23) si les rafales (36) générées par l'unité de stimulation (11) ne réinitialisent pas la phase de l'activité neuronale des neurones respectivement stimulés,
- l'unité de commande (10), après qu'il a été déterminé que les rafales (36) réinitialisent la phase d'activité neuronale des neurones stimulés respectifs, commande l'unité de stimulation (11) de telle manière que les rafales (36) générées ensuite par l'unité de stimulation (11) ont une intensité réduite de stimulation, et que
- la réduction de l'intensité de stimulation des rafales (36) générées par l'unité de stimulation (11) est poursuivie jusqu'à ce que l'unité de commande (10) détermine que les rafales (36) ne réinitialisent pas la phase d'activité neuronale des neurones stimulés respectifs.

**2.** Le dispositif (2) d'après la revendication 1, sachant que les stimuli (22) sont des stimuli acoustiques, visuels, tactiles, vibratoires, thermiques, olfactifs, gustatifs, transcutanés électriques, transcutanés magnétiques, transcrâniens électriques et/ou transcrâniens magnétiques et/ou à ultrasons.

**3.** Le dispositif (2) d'après la revendication 1 ou 2, sachant que deux rafales (36) qui se succèdent dans le temps et qui sont générées respectivement dans différents canaux de stimulation (12 - 15) sont décalées dans le temps l'une par rapport à l'autre par $T_{stim}/S$, sachant que $T_{stim} = 1/f_{stim}$ s'applique et que $f_{stim}$ est une fréquence comprise entre 1 et 30 Hz et que S est le nombre de canaux de stimulation (12 - 15).

4. Le dispositif (2) d'après la revendication 1 ou 2, sachant que l'unité de stimulation (11) génère les rafales (36) dans une trame temporelle constituée de cycles successifs, et que les cycles ont chacun une durée $T_{stim}$, sachant que $T_{stim} = 1/f_{stim}$ s'applique et $f_{stim}$ est une fréquence comprise entre 1 et 30 Hz.

5. Le dispositif (2) d'après la revendication 4, sachant que l'unité de stimulation (11) génère exactement une rafale (36) dans chaque canal de stimulation (12 - 15) dans un des cycles.

6. Le dispositif (2) d'après la revendication 4 ou 5, sachant que chacun des cycles est subdivisé en S sous-cycles, notamment en S sous-cycles égaux, et que l'unité de stimulation (11) dans chaque sous-cycle ne génère pas plus d'une rafale (36), le nombre des canaux de stimulation (12 - 15) étant S.

7. Le dispositif (2) d'après l'une quelconque des revendications de 4 à 6, sachant que l'unité de stimulation (11) produit des rafales (36) pendant n cycles successifs et ne produit pas de stimuli pendant les m cycles suivants et que ledit motif est poursuivi périodiquement, sachant que n et m sont des nombres entiers non négatifs.

8. Le dispositif (2) d'après l'une quelconque des revendications de 4 à 7, les rafales (36) étant adaptées pour supprimer l'activité pathologiquement synchrone et oscillatoire de la population neuronale (31) lorsqu'elles sont administrées au patient via la pluralité de canaux de stimulation (12 - 15).

9. Le dispositif (2) d'après la revendication 8, sachant que la durée $T_{stim}$ d'un cycle correspond essentiellement à la période moyenne d'oscillation pathologique de la population neuronale (31).

10. Le dispositif (2) d'après l'une des revendications précédentes, sachant que l'intensité de stimulation d'une rafale (36) est réduite en réduisant l'amplitude et/ou le nombre de stimuli (37) contenus dans la rafale (36).

11. Le dispositif (2) d'après l'une des revendications précédentes, sachant que, pour tester si les rafales (36) générées par l'unité de stimulation (11) ne réinitialisent pas la phase d'activité neuronale des neurones stimulés, une pluralité de rafales identiques (36) est générée séquentiellement et l'unité de commande (10) détermine l'activité neuronale enregistrée par l'unité de mesure (16) en réponse à l'application de chacune de la pluralité de rafales (36) et détermine que les rafales (36) ne réinitialisent pas la phase d'activité neuronale des neurones stimulés si l'activité neuronale moyenne sur tous les signaux de réponse est égale ou égale à zéro.

12. Le dispositif (2) d'après l'une quelconque des revendications de 1 à 10, sachant que pour tester si les rafales (36) générées par l'unité de stimulation (11) ne réinitialisent pas la phase d'activité neuronale des neurones stimulés, une pluralité de rafales identiques (36) est générée séquentiellement et l'unité de commande (10) détermine la phase d'activité neuronale reçue par l'unité de mesure (16) en réponse à l'application de chacune de la pluralité de rafales (36) et détermine que les rafales (36) ne réinitialisent pas la phase d'activité neuronale des neurones stimulés si la distribution des phases ne présente aucune valeur d'accumulation.

13. Un procédé de stimulation des neurones (31), sachant que

- une unité de stimulation non-invasive (11) génère des stimuli (22) dans une pluralité de canaux de stimulation (12 - 15), sachant que
- les stimuli (22) stimulent une population de neurones (31) dans le cerveau et/ou dans la moelle épinière (30) d'un patient via les canaux de stimulation (12 - 15) à des endroits respectivement différents,
- des signaux de mesure (23) qui représentent une activité neuronale de la population neuronale (31) stimulée par les stimuli (22),
- l'unité de stimulation (11) génère de manière répétitive des rafales (36), chacune comprenant une pluralité de stimuli (37), dans chacun des canaux de stimulation (12 - 15),
- les rafales (36) générées dans différents canaux de stimulation (12 - 15) sont décalées dans le temps les unes par rapport aux autres,
- les rafales (36) ne réinitialisent pas la phase d'activité neuronale des neurones stimulés respectifs,
- on vérifie à l'aide des signaux de mesure (23) si les rafales (36) générées par l'unité de stimulation (11) ne réinitialisent pas la phase d'activité neuronale des neurones stimulés respectifs,
- après qu'il a été déterminé que les rafales (36) réinitialisent la phase d'activité neuronale des neurones stimulés respectifs, l'unité de stimulation (11) produit ensuite des rafales (36) ayant une intensité de stimulus réduite, et que

- la réduction de l'intensité de stimulation des rafales (36) générées par l'unité de stimulation (11) est poursuivie jusqu'à ce qu'il soit déterminé que les rafales (36) ne réinitialisent pas la phase d'activité neuronale des neurones stimulés respectifs.

14. Un logiciel destiné à être exécuté dans un système de traitement de données, le logiciel

- générant des signaux de commande pour commander une unité de stimulation non-invasive (11), sachant que l'unité de stimulation (11) génère des stimuli (22) dans une pluralité de canaux de stimulation (12 - 15) et que les stimuli (22) stimulent une population neuronale (31) dans le cerveau et/ou dans la moelle épinière (30) d'un patient via les canaux de stimulation (12 - 15) à différents endroits, et sachant que des signaux de mesure (23) sont respectivement enregistrés, qui représentent une activité neuronale de la population de neurones (31) stimulée par les stimuli (22), sachant que les signaux de commande commandent l'unité de stimulation (11) de manière que

- l'unité de stimulation (11) génère de manière répétitive des rafales (36), chacune comprenant une pluralité de stimuli (37), dans chacun des canaux de stimulation (12 - 15),

- les rafales (36) générées dans différents canaux de stimulation (12 - 15) sont décalées dans le temps les unes par rapport aux autres,

- les rafales (36) ne réinitialisent pas la phase d'activité neuronale des neurones stimulés respectifs,

- on vérifie à l'aide des signaux de mesure (23) si des rafales (36) générées par l'unité de stimulation (11) ne réinitialisent pas la phase d'activité neuronale des neurones stimulés respectifs,

- après qu'il a été déterminé que les rafales (36) réinitialisent la phase d'activité neuronale des neurones stimulés respectifs, l'unité de stimulation (11) produit ensuite des rafales (36) ayant une intensité de stimulus réduite, et

- la réduction de l'intensité de stimulation des rafales (36) générées par l'unité de stimulation (11) est poursuivie jusqu'à ce qu'il soit déterminé que les rafales (36) ne réinitialisent pas la phase d'activité neuronale des neurones stimulés respectifs.

Fig. 1

Fig. 2

EP 3 188 794 B1

Fig. 3

Fig. 4A

Fig. 4B

Fig. 5

Fig.6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 102012002436 A1

### In der Beschreibung aufgeführte Nicht-Patentliteratur

- **P. A. TASS ; I. ADAMCHIC ; H.-J. FREUND ; T. VON STACKELBERG ; C. HAUPTMANN.** Counteracting tinnitus by acoustic coordinated reset neuromodulation. *Restorative Neurology and Neuroscience,* 2012, vol. 30, 137-159
- **P. A. TASS ; I. ADAMCHIC ; H.-J. FREUND ; T. VON STACKELBERG ; C.HAUPTMANN.** Counteracting tinnitus by acoustic coordinated reset neuromodulation. *Restorative Neurology and Neuroscience,* 2012, vol. 30, 137-159
- **I. ADAMCHIC ; T. TOTH ; C. HAUPTMANN ; P. A. TASS.** Reversing patho-logically increased EEG power by acoustic CR neuromodulation. *Human Brain Mapping,* 2014, vol. 35 (2099-21), 18
- **A. N. SILCHENKO ; I. ADAMCHIC ; C. HAUPTMANN ; P. A. TASS.** Impact of acoustic coordinated reset neuromodulation on effective Connectivity in a neural network of phantom sound. *Neuroimage,* 2013, vol. 77, 133-147
- **I. ADAMCHIC ; B. LANGGUTH ; C. HAUPTMANN ; P. A. TASS.** Abnormal brain activity and cross-frequency cou-pling in the tinnitus network. *Frontiers in Neuroscience,* 2014, vol. 8, 284
- *Phys. Rev. E,* 2004, vol. 69, 051909-1, 24
- **P. A. TASS.** Transmission of stimulus-locked responses in two coupled phase oscillators. *Phys. Rev. E,* 2004, vol. 69, 051909-1, 24
- **N. E. HUANG et al.** The empirical mode decomposition and the Hilbert spectrum for nonlinear and non-stationary time series analysis. *Proc. R. Soc. A: Math. Phys. Eng. Sci.,* 1998, vol. 454, 903-995
- **N. E. HUANG et al.** A confidence limit for the empirical mode decomposition and Hilbert spectral analysis. *Proceedings of the Royal Society of London Series A,* 2003, vol. 459, 2317-2345